# EUROPÄISCHE PATENTSCHRIFT

(11) **EP 0 580 624 B1**
(45) Veröffentlichungstag und Bekanntmachung des Hinweises auf die Patenterteilung: **16.07.1997**
(21) Anmeldenummer: 92907196.7
(22) Anmeldetag: 31.03.1992
(51) Int. Cl.: C07H 19/09, A61K 31/70

(54) **NEUE CYTARABIN-DERIVATE, IHRE HERSTELLUNG UND VERWENDUNG**
NOVEL CYTARABINE DERIVATIVES, THEIR PRODUCTION AND USE
NOUVEAUX DERIVES DE LA CYTARABINE, PREPARATION ET UTILISATION

(30) Priorität: 10.04.1991 DE 4111679
(43) Veröffentlichungstag der Anmeldung: 02.02.1994
(73) Patentinhaber: SCHOTT, Herbert, D-72076 Tübingen (DE)
(72) Erfinder: KLUGE, Michael, D-6701 Kallstadt (DE); SCHOTT, Herbert, D-7400 Tuebingen (DE)
(74) Vertreter: Kinzebach, Werner, Dr.
(86) Internationale Anmeldenummer: EP9200706
(87) Internationale Veröffentlichungsnummer: WO9218519

(56) Entgegenhaltungen:
- BIOCHIMICA ET BIOPHYSICA ACTA Bd. 1026, 1990, AMSTERDAM, NL, Seiten 69-79, R.A. SCHWENDENER et al.
- CANCER DRUG DELIVERY Bd. 3, Nr. 2, 1986, NEW YORK, US, Seiten 123-129, R.A. SCHWENDENER

## Beschreibung

Die vorliegende Erfindung betrifft neue Cytarabin-Derivate, deren Herstellung sowie deren Verwendung bei der Bekämpfung von Krankheiten.

AraC (= Cytarabin = 4-Amino-1-β-D-arabinofuranosyl-2(1H)-pyrimidinon bzw. 1-β-D-Arabinofuranosylcytosin, Merck-Index 11. Auflage, Nr. 2790) ist ein in der Chemotherapie von Krebserkrankungen bewährtes Zytostatikum. Durch die im Körper anwesende Cytosindesaminase wird das AraC jedoch schnell desaminiert und damit unwirksam. Zur Erzielung einer therapeutischen Wirkung muß es daher in hohen Dosen appliziert werden, die für den Patienten mit unangenehmen Nebenwirkungen verbunden sind.

Um die zu schnelle enzymatische Desaminierung zeitlich zu verzögern, wurde die Aminogruppe des Cytosinrestes bisher mit Acyl-Schutzgruppen maskiert (Int. J. Cancer 37, 149 (1986)). Die hierbei erhaltenen N⁴-AcylAraC-Derivate zeigten aber, auch wenn sie in Form von Liposomen appliziert wurden, im Vergleich zu underivatisiertem AraC keine verbesserte zytostatische Wirkung. Die N⁴-Acylamidbindung dieser AraC-Prodrugs konnte die enzymatische Desaminierung in vivo nur kurzfristig verzögern.

Außerdem sind aus dem Stand der Technik AraC-Derivate bekannt, deren Aminogruppe mit Oleyl- bzw. Hexadecyl-Schutzgruppen (Biochimica et Biophysica Acta 1026, 69 (1990); Cancer and Drug Delivery, vol. 3 (2), 123 (1986)) maskiert ist.

Es wurden nun neue Cytarabin-Derivate gefunden, in denen das Cytarabin effektiver gegen eine enzymatische Desaminierung geschützt ist.

Gegenstand der Erfindung sind Cytarabin-Derivate der Formel I 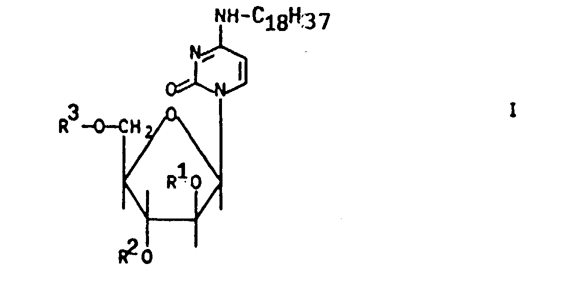 worin
- R¹, R² und R³,: die gleich oder verschieden sein können, Wasserstoffatome, einen aliphatischen C₂₋₅-Acylrest, eine Benzoylgruppe oder einen Carboxy-C₁₋₃-alkylencarbonylrest
bedeuten.

Als Säurerest ist für R¹, R² und R³ insbesondere der Bernsteinsäurerest zu nennen.

Die neuen Cytarabin-Derivate der Formel I lassen sich herstellen, indem man eine Verbindung der Formel II 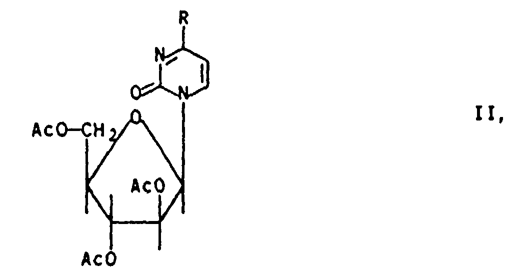 worin Ac eine Acetylgruppe und R ein Chloratom oder der Rest 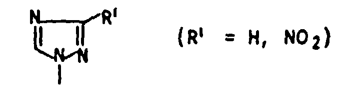 darstellen, mit Octadecylamin
umsetzt und in den so erhaltenen Verbindungen qegebenenfalls anschlieβend Acetylreste gegen R¹, R² bzw. R³ austauscht.

Die Umsetzung gelingt gut in einem polaren Lösungsmittel wie Dioxan oder Methanol in der Siedehitze.

Der Austausch der Acetylreste gegen Wasserstoffatome bzw. andere Acylreste kann wie folgt durchgeführt werden:

Die Acetylreste werden durch Ammonolyse in wäßrigem oder methanolischem Ammoniak durch Stehen bei 20 bis 60°C innerhalb von 2 bis 24 Stunden gegen Wasserstoffatome ausgetauscht. In den so erhaltenen Verbindungen werden durch Umsetzung mit entsprechenden Carbonsäureanhydriden oder Carbonsäurechloriden die Wasserstoffatome anschließende gegen Acylreste ausgetauscht.

Die Umsetzung gelingt gut in Pyridin bei Raumtemperatur.

Die für die Umsetzung benötigten Ausgangsmaterialien sind bekannte Stoffe (vgl. J.C.S. Perkin I 1982, 1171) oder lassen sich nach bekannten Verfahren herstellen.

Die neuen Verbindungen zeichnen sich gegenüber dem AraC durch eine bessere Desaminaseresistenz aus. Sie lassen sich aufgrund ihrer amphiphilen Eigenschaften leicht zu wäßrigen galenischen Applikationsformen verarbeiten.

Weiter können durch die Anzahl, Länge, Art und Lage der jeweiligen Substituenten in den erfindungsgemäßen AraC-Derivaten die amphiphilen Eigenschaften überraschenderweise in weiten Grenzen variiert werden. Die amphiphilen AraC-Derivate sind somit in wäßrigen Puffersystemen löslich und/oder in Form von Liposomen dispergierbar. Zur Liposomenbildung können alle an sich bekannten Verfahren der Liposomendarstellung verwendet werden wie beispielsweise Ultraschall, Gelchromatographie, Detergenzdialyse. Die jeweils eingeführten lipophilen Reste beeinflussen außerdem maßgeblich die Größe und Stabilität der Liposomen, die sich aus den jeweiligen amphiphilen AraC-Derivaten zusammen mit weiteren Lipidkomponenten bilden.

Durch die gezielte Einführung von lipophilen Resten läßt sich aber nicht nur der amphiphile Charakter der erfindungsgemäßen AraC-Derivate gezielt steuern, sondern überraschenderweise auch die zytostatische Wirkung von AraC entscheidend optimieren.

Die neuen Verbindungen lassen sich wie das AraC selbst gegen maligne Erkrankungen der blutbildenden Zellen einsetzen, insbesondere gegen akute Leukämien und chronisch myeloische Leukämie im Blastenschub.

Die zytostatische Wirkung der amphiphilen AraC-Derivate läßt sich in sogenannten Immunoliposomen überraschenderweise zur gezielten Zerstörung von bestimmten Tumorzellen nutzen. Hierzu werden die amphiphilen AraC-Derivate zusammen mit weiteren funktionalisierten Lipidkomponenten in Form von Liposomen in physiologischen Puffersystemen dispergiert. An funktionellen Gruppen der Liposomenmembran werden monoklonale Antikörper immobilisiert. Die so erhaltenen Immunoliposomen werden in vitro bevorzugt von den Tumorzellen aufgenommen, die das dem Antikörper entsprechende Antigen exprimieren. Die Folge dieses Zell-targetings ist die selektive Zerstörung der jeweiligen Zieltumorzelle in einem Gemisch verschiedener Zellen.

Die Wirkung der neuen Verbindungen läßt sich in folgender Versuchsanordnung zeigen:

DBA/2-Mäusen wurden L1210 Tumorzellen intravenös injiziert, wodurch eine Leukämie simuliert wurde. Am Tag 3 und 7 nach der Tumorzell-Injektion wurden den tumortragenden Tieren verschiedene Dosierungen der Testsubstanz oder Lösungsmittel verabreicht. Insgesamt ergab sich folgende Einteilung in die einzelnen Versuchsgruppen:
- Kontrollgruppe (Lösungsmittel)
- i.v. Applikation (2 Dosierungen)
- i.p. Applikation (2 Dosierungen)
- i.v. AraC als Referenz (2 Dosierungen)
- i.p. AraC als Referenz (2 Dosierungen)

Als Parameter für den Therapieerfolg wird die mediane überlebenszeit der einzelnen Versuchsgruppen (10 Tiere pro Gruppe) berechnet.

In diesen Versuchen zeigten die neuen Verbindungen eine bessere Wirkung als AraC.

Die neuen Verbindungen sollen in einer Dosierung von etwa 40 - 1000 mg pro Patient und Tag eingesetzt werden.

### Beispiel 1

### Darstellung von 4-(1-0ctadecylamino)-1-β-D-arabinofuranosyl-2(1H)-pyrimidinon

60 g (142 mmol) 4-(1,2,4-Triazol-1-yl)-1-β-D-2',3',5'-tri-o-acetylarabinofuranosyl-2(1H)-pyrimidinon wurden in 450 ml trockenem Dioxan gelöst. Zu dieser Lösung wurde eine Lösung von 59 g (221 mmol) 1-Octadecylamin, gelöst in 450 ml Ethanol zugetropft. Anschließend wurde der Ansatz 2 h unter Rückfluß erhitzt. Das Lösungsmittel des erkalteten Reaktionsansatzes wurde im Vakuum abgezogen und der verbleibende Sirup mit 750 ml Methanol, das mit Ammoniak gesättigt war, gelöst. Die Lösung wurde ca. 12 h bei Raumtemperatur belassen. Hierbei fiel das gewünschte Produkt nach und nach aus. Zur vollständigen Kristallisation wurde der Ansatz einige Zeit bei -20°C aufbewahrt. Dann wurde der Niederschlag abgesaugt, mit Diethylether nachgewaschen und bei ca. 60°C im Trockenschrank getrocknet. Das erhaltene Rohprodukt (ca. 60 bis 70 g) wurde aus 80 %igem wäßrigen Methanol umkristallisiert. Hierbei wurden 70 g 4-(1-Octadecylamino)-1-β-D-arabinofuranosyl-2(1H)-pyrimidinon als weißes kristallines Produkt erhalten, das auf der Kieselgelplatte im System Chloroform/Methanol (4:1; v:v) einen R_{f}-Wert von 0,43 aufwies.

### Beispiel 2

### Darstellung von 4-(1-Octadecylamino)-1-β-D-5'-0-succinoylarabinofuranosyl-2(1H)-pyrimidinon

2,3 g (4,3 mmol) 4-(1-Octadecylamino)-1-β-D-arabinofuranosyl-2(1H)-pyrimidinon wurden in 10 ml trockenem Pyridin gelöst, mit 0,7 g (5,7 mmol) 4-Dimethylaminopyridin und 0,7 g (7 mmol) Bernsteinsäureanhydrid versetzt. Nach ca. 12 h Rühren bei Raumtemperatur wurde der Reaktionsansatz an einer Kationenaustauschersäule (LEWATIT, Pyridiniumform) mit Pyridin/Wasser (1:4; v:v) chromatographiert. Das Eluat wurde im Vakuun zum sirup konzentriert, der anschließend mehrmals mit Toluol abrotiert wurde. Der erhaltene Schaum wurde in wenig Chloroform gelöst und an einer Kieselgelsäule mit Chloroform/Ethanol-Gemischen fraktioniert, wobei der Ethanolanteil stufenweise von 10 % auf 100 % gesteigert wurde. Die Fraktionen des gewünschten Produkts, das bei hohem Ethanolanteil die Säule verließ, wurden vereinigt und im Vakuum zum Sirup konzentriert. Der Sirup wurde in Chloroform gelöst und aus einem überschuß eines Ether/Hexan-Gemisches (3:2; V:V) gefällt. Durch HPLC an einer C₁₈-Umkehrphase konnten die 2',3'-Isomere entfernt werden, die als Nebenprodukte auftraten. Fraktionen, die das gewünschte Produkt enthielten, wurden konzentriert. Hierbei wurde 4-(1-Octadecylamino)-1-β-D-5'-D-succinoyl-arabinofuranosyl-2(1H)-pyrimidinon als weißes Pulver erhalten, das auf der Kieselgelplatte im System Chloroform/Ethanol (4:1; v:v) einen R_{f}-Wert von 0,47 aufwies.

### Beispiel 3

### Darstellung der Liposomenpräparate

Für die Herstellung der Liposomendispersion wurden pro ml Chloroform-Methanol (1:1; v:v) jeweils 100 mg Soja-Phosphatidylcholin, 10 mg Cholesterol, 1 mg α-Tocopherol, 7 mg N²-Palmitoyl-N⁶-succinoyl-L-lysin und 12 mg 4-(1-Octadecylamino)-1-β-D-arabinofuranosyl-2(1H)pyrimidinon gelöst. 0,6 ml dieser Lipidstammlösung wurden in einem Reagenzglas durch Verblasen mit Luft in einen Lipidfilm überführt, der anschließend ca. 1 h bei 50°C im Vakuum getrocknet wurde. Der Lipidfilm wurde mit 3 ml 10 mM PBS (0,9 % NaCl und 10 mM NaH₂PO₄, pH 7,3) versetzt und mit Hilfe einer Mikrospitze eines Desintegrators 30 min mit 40 Watt beschallt. Hierbei bildete sich eine opaleszente Liposomendispersion, die für die folgende Reaktion verwendet wurde.

### Beispiel 4

### Darstellung der Immunoliposomen

1,2 nmol eines Antikörpers wurden als Lyophilisat mit 50 µl des Liposomenpräparats aus Beispiel 3 und 7 mg (27 µmol) N(3-Dimethylaminopropyl)-N'-ethyl-carbodiimid X HCl (EDC) versetzt und durch Zugabe von 30 µl PBS (pH 1) auf pH 4 eingestellt. Im einstündigen Abstand wurden noch zweimal Jeweils 7 mg EDC zugegeben. Nach ca. 5 h Rühren bei Raumtemperatur wurde der Reaktionsansatz auf eine ULTROGEL ACA 22-Säule aufgetragen und mit PBS (pH 7,4) fraktioniert. Fraktionen deren Absorptionsverhältnisse mit den Werten des eingesetzten Liposomenpräparats übereinstimmen, wurden vereinigt und zum hell-targeting verwendet.

## Patentansprüche

1. Cytarabin-Derivate der Formel I 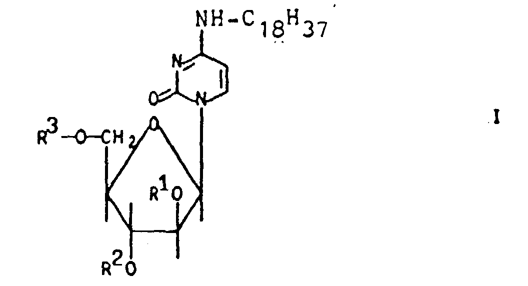 worin
R¹, R² und R³, die gleich oder verschieden sein können, Wasserstoffatome, einen aliphatischen C₂₋₅-Acylrest eine Benzoylgruppe oder einen Carboxy-C₁₋₃-alkylencarbonylrest
bedeuten.

2. Verfahren zur Herstellung der Cytarabin-Derivate der Formel I gemäß Anspruch 1, dadurch gekennzeichnet, daß man eine Verbindung der Formel II 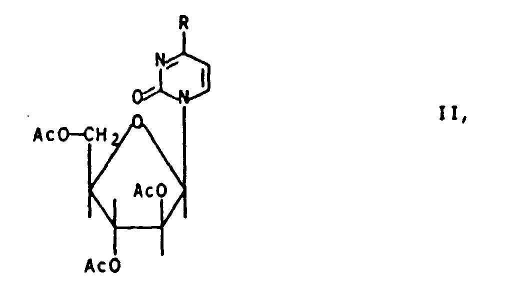 worin Ac eine Acetylgruppe und R ein Chloratom oder der Rest 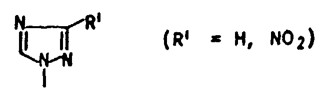 darstellen, mit Octadecylamin
umsetzt und in den so erhalenen Verbindungen gegebenenfalls Acetylreste gegen R¹, R² bzw. R³ austauscht.

3. Imnunoliposomen enthaltend ein Cytarabin-Derivat der Formel I gemäß Anspruch 1.

4. Cytarabin-Derivate der Formel I gemäß Anspruch 1 zur Verwendung bei der Bekämpfung von Krankheiten.

## Claims

1. Cytarabine derivatives of formula I 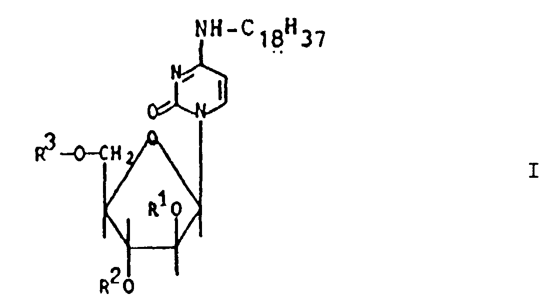 wherein
R¹, R² and R³, which may be identical or different, denote hydrogen atoms, an aliphatic C₂₋₅-acyl group, a benzoyl group or a carboxy-C₁₋₃-alkylene carbonyl group.

2. Process for preparating the cytarabine derivatives of formula I according to claim 1, characterised in that a compound of formula II 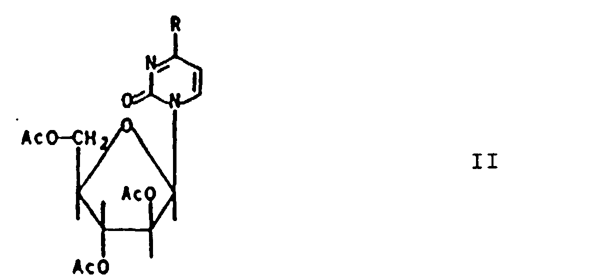 wherein Ac is an acetyl group and R denotes a chlorine atom or the group 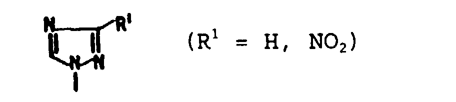 is reacted with octadecylamine and in the resulting compounds acetyl groups are optionally replaced by R¹, R² or R³.

3. Immunoliposomes containing a cytarabine derivative of formula I according to claim 1.

4. Cytarabine derivatives of formula I according to claim 1 for use in fighting diseases.

## Revendications

1. Dérivés de la cytarabine de formule I 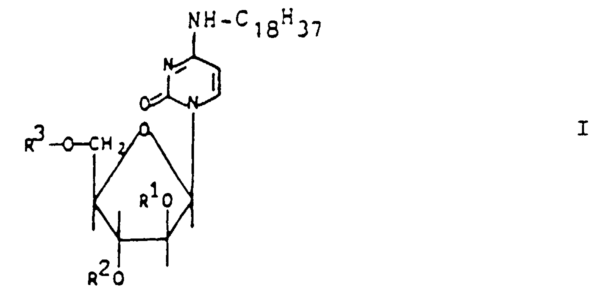 dans laquelle R¹, R² et R³, qui peuvent être identiques ou différents, représentent des atomes d'hydrogène, un radical aliphatique acyle en C₂-C₅, un groupe benzoyle ou un radical carboxy-alkylènecarbonyle en C₁-C₃.

2. Procédé de préparation des dérivés de la cytarabine de formule I selon la revendication 1, caractérisé en ce que l'on fait réagir avec de l'octadécylamine un composé de formule II 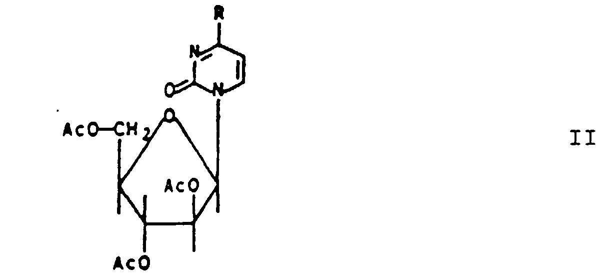 dans laquelle Ac est un groupe acétyle et R un atome de chlore ou bien le radical 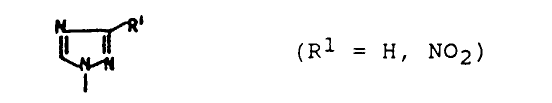 et on remplace, le cas échéant, dans les composés ainsi obtenus les radicaux acétyle par R¹, R² ou R³.

3. Immunoliposomes contenant un dérivé de la cytarabine de formule I selon la revendication 1.

4. Dérivés de la cytarabine de formule I selon la revendication 1 destinés à l'utilisation dans le traitement de maladies.
